# FASCICULE DE BREVET EUROPEEN

(11) **EP 3 548 098 B1**
(45) Date de publication et mention de la délivrance du brevet: **05.08.2020**
(21) Numéro de dépôt: 17816983.5
(22) Date de dépôt: 04.12.2017
(51) Int. Cl.: A61K 49/08, B82Y 5/00, A61K 49/18

(54) **MATÉRIAU COEUR ORGANIQUE MAGNÉTIQUE-ÉCORCE INORGANIQUE, SON PROCÉDÉ DE PRÉPARATION ET SES UTILISATIONS POUR LA DÉLIVRANCE MAGNÉTO-STIMULÉE DE SUBSTANCES D'INTÉRÊT**
MAGNETISCHES MATERIAL MIT ORGANISCHEM KERN UND ANORGANISCHER HÜLLE, HERSTELLUNGSVERFAHREN DAFÜR UND VERWENDUNGEN DAVON ZUR MAGNETISCH STIMULIERTEN FREISETZUNG VON SUBSTANZEN VON INTERESSE
MAGNETIC ORGANIC CORE-INORGANIC SHELL MATERIAL, PRODUCTION METHOD THEREOF AND USES OF SAME FOR THE MAGNETICALLY STIMULATED DELIVERY OF SUBSTANCES OF INTEREST

(30) Priorité: 05.12.2016 FR 1661952
(43) Date de publication de la demande: 09.10.2019
(73) Titulaire: Centre National de la Recherche Scientifique, 75016 Paris (FR); Université de Bordeaux, 33000 Bordeaux (FR); Institut Polytechnique de Bordeaux, 33402 Talence (FR)
(72) Inventeur: BACKOV, Rénal, 33200 Bordeaux-Cauderan (FR); SCHMITT, Véronique, 33400 Talence (FR); BAILLOT, Marion, 33000 Bordeaux (FR); SANDRE, Olivier, 33600 Pessac (FR)
(74) Mandataire: Goulard, Sophie
(86) Numéro de dépôt international: PCT/FR2017/053378
(87) Numéro de publication internationale: WO 2018/104642

(56) Documents cités:
- EP-A1- 2 990 382
- WO-A1-2011/012813

## Description

La présente invention est relative à un matériau sub-micrométrique constitué d'une enveloppe de silice renfermant un cœur de cire superparamagnétique, à son procédé de préparation et à ses utilisations, notamment pour la délivrance magnéto-stimulée de substances d'intérêt.

Il est connu d'encapsuler des molécules d'intérêt telles que des médicaments, des colorants, des pigments, des réactifs, des parfums, des pesticides, etc..., pour les protéger des agressions extérieures, notamment de l'oxydation, pour les acheminer vers un lieu d'administration où elles pourront être délivrées ou bien encore pour les stocker avant une utilisation dans des conditions où elles seront libérées de leur capsule sous l'influence d'un stimulus interne ou externe. Une des premières applications de la microencapsulation a été la mise au point d'un papier copiant sans carbone commercialisé à la fin des années 60, dans lequel des microcapsules emprisonnant une encre étaient présentes sur le verso d'une feuille de papier de façon à libérer l'encre par rupture des capsules sous la pression exercée par la pointe d'un stylo lors de l'écriture. De nos jours, l'encapsulation se développe dans différents secteurs industriels tels que les industries pharmaceutique, cosmétique, alimentaire, textile et agricole. Les capsules et microcapsules deviennent de plus en plus sophistiquées, notamment dans le domaine pharmaceutique où elles permettent de délivrer de façon contrôlée et/ou ciblée un ou plusieurs principes actifs.

Différents types et morphologies de capsules ont déjà été proposés tels que par exemple des capsules protéiques, des capsules peptidiques, des cyclodextrines, des liposomes thermosensibles, des polymérosomes, des colloïdosomes, des microcapsules à enveloppes de silice, des nanocapsules comprenant un cœur en silice et une enveloppe en polymère thermosensible tel que le poly(*N*-isopropylacrylamide) (PNIPAM), ou à l'inverse un cœur de polymère thermosensible tel que le Pluronic® F68 / poly(alcool vinylique) et une coquille de silice, des microsphères d'hydrogel thermosensible, des microsphères de PNIPAM-Polylactide, etc.... De nombreuses méthodes permettant de préparer ces différents types et morphologies de capsules ont également été développées durant ces dernières années, telles que par exemple et de façon non exhaustive, la précipitation de polymères par séparation de phase, le dépôt d'électrolyte couche par couche, la polymérisation par polycondensation interfaciale, etc.... Selon le type et la morphologie des capsules développées, la libération des molécules d'intérêt peut être lente et progressive (c'est-à-dire prolongée dans le temps) ou provoquée (c'est-à-dire déclenchée par une action). En particulier, la libération des molécules d'intérêt peut être déclenchée sous l'effet d'un ou plusieurs stimulus intérieurs et/ou extérieurs tels que par exemple un changement de pH, un procédé rédox, une catalyse enzymatique, des ultrasons, l'utilisation d'agents spécifiques tels que des agents moussants, un changement de température, une irradiation lumineuse, des rayonnements proches infrarouges, une modification de la pression osmotique, la perturbation de l'enrobage par un gonflement du noyau, un champ électrique ou un champ magnétique.

En particulier, la demande de brevet FR-A-2 948 581 décrit un matériau micrométrique (12,5-50 µm) constitué d'une enveloppe de silice renfermant un cœur de cire contenant une ou plusieurs substances d'intérêt, ces matériaux étant préparés par minéralisation d'une émulsion de Pickering, c'est-à-dire d'une émulsion de type huile-dans-eau dans laquelle la dispersion des gouttelettes d'huile dans l'eau est stabilisée par des nanoparticules colloïdales adsorbées à l'interface eau/huile. En utilisant une huile cristallisable, c'est-à-dire une huile dont la température de fusion (T_{F}) est assez basse (par exemple 37°C), il est possible de préparer un matériau dans lequel la phase encapsulée (i.e. le cœur) est solide à température ambiante mais devient liquide lorsque l'on chauffe ledit matériau à l'aide d'une platine chauffante à une température de l'ordre de 50-60°C, provoquant ainsi la rupture de la capsule par fusion et expansion thermique de la phase encapsulée et la libération concomitante et rapide de la ou des substances d'intérêt contenues dans la phase encapsulée.

Toutefois, ce matériau est de taille micrométrique, ce qui ne permet pas de l'utiliser dans certains domaines d'application tels que le domaine des nano-médicaments (e.g. injection intraveineuse de nano-médicaments) ou des nano-cosmétiques (e.g. vésicules ou capsules renfermant un parfum, une vitamine, un antioxydant). Par ailleurs, l'utilisation d'une source de chaleur externe macroscopique (e.g. platine chauffante) n'est pas adaptée pour la libération des molécules d'intérêt dans des applications *in vivo.* De plus, les températures et les vitesses de chauffe employées pour permettre la rupture de l'enveloppe de silice sont trop élevées pour des applications *in vivo* et/ou certains environnements sensibles à la chaleur. De plus, elles sont consommatrices d'énergie. En particulier, l'enveloppe de silice est un excellent isolant thermique, il est donc difficile de passer l'enceinte adiabatique que constitue ladite enveloppe, notamment par chauffage externe. Enfin, le contrôle de la libération des molécules d'intérêt n'est pas optimisé (e.g. libération trop rapide).

Il n'existe donc pas à ce jour de système sub-micrométrique autorisant une libération progressive de molécules d'intérêt sous l'effet d'un stimulus intérieur ou extérieur en conditions douces, tout en garantissant un meilleur contrôle de la libération, notamment pour des applications *in vivo.*

Le brevet EP 2990382 A1 décrit également des nanoparticules d'oxyde de fer capables d'absorber des ondes électromagnétiques ainsi que des films minces les contenant.

Le but de la présente invention est donc de proposer une capsule sub-micrométrique permettant d'encapsuler une ou plusieurs molécules d'intérêt qui puisse(nt) être libérée(s) progressivement en conditions douces, tout en garantissant un meilleur contrôle de la libération, notamment pour des applications *in vivo.*

Le but de la présente invention est également de proposer un procédé de préparation de capsules sub-micrométriques facile à mettre en œuvre et économique, ledit procédé permettant d'encapsuler une ou plusieurs molécules d'intérêt qui puisse(nt) être libérée(s) de façon progressive en conditions douces, tout en garantissant un meilleur contrôle de la libération, notamment pour des applications *in vivo.*

La présente invention a pour premier objet un matériau sous la forme de particules solides contenant une phase grasse solide à la température de stockage dudit matériau et une enveloppe continue comprenant au moins un oxyde de silicium et emprisonnant ladite phase grasse, ladite phase grasse comprenant une huile cristallisable ayant une température de fusion (T_{F}) inférieure à 100°C environ et au moins une substance d'intérêt, ledit matériau étant caractérisé en ce qu'il est sub-micrométrique et en ce que la phase grasse comprend en outre des nanoparticules superparamagnétiques fonctionnalisées en surface par au moins un acide gras.

Selon la présente invention, on entend par « température de stockage dudit matériau », la température à laquelle le matériau conforme à la présente invention est conservé avant son utilisation. Cette température est toujours inférieure au point de fusion de l'huile cristallisable contenue dans la phase grasse. Elle est de préférence comprise entre -25 et 25°C environ, et de préférence encore entre 0 et 22°C environ.

Lorsqu'on soumet ledit matériau à un champ magnétique alternatif, les nanoparticules superparamagnétiques fonctionnalisées contenues dans la phase grasse s'échauffent localement, ce qui entraîne localement le chauffage de la phase grasse à une température supérieure à la température de fusion de l'huile cristallisable (T_{F}). On observe alors une expansion thermique de la phase grasse entraînant la rupture de l'enveloppe de silice et la libération progressive de la phase grasse en fusion (c'est-à-dire à l'état liquide) comprenant la ou les substances d'intérêt. Par ailleurs, l'échauffement local des nanoparticules superparamagnétiques fonctionnalisées est suffisant pour permettre la fonte et/ou la fusion de la phase grasse entraînant ainsi la libération de la ou des substances d'intérêt.

On entend dans le cadre de cet exposé par le terme « huile cristallisable », les matières grasses et les mélanges de matières grasses, d'origine naturelle (animale ou végétale) ou synthétique, dont le point de fusion est supérieur à 15°C environ, de préférence dont le point de fusion varie de 20 à 100°C environ, et en particulier de 20 à 50°C environ. Tous les points de fusion mentionnés dans la description de la présente demande font référence à des points de fusion déterminés par calorimétrie différentielle à balayage à pression atmosphérique (bien connue sous l'anglicisme « *Differential Scanning Calorimetry* » (DSC)).

L'huile cristallisable forme une partie majoritaire de la phase grasse et peut même, outre la ou les substances d'intérêt et les nanoparticules superparamagnétiques fonctionnalisées, être l'unique constituant de celle-ci. Généralement, l'huile cristallisable représente au moins 50% en masse environ, de préférence de 50 à 99,8% en masse environ, et de préférence encore de 75 à 98% en masse environ de la phase grasse.

Le choix de l'huile cristallisable dépend naturellement de l'application envisagée pour le matériau et donc de la température à laquelle on souhaite observer l'expansion thermique de la phase grasse et par voie de conséquence la rupture de l'enveloppe de silice. Parmi les huiles cristallisables utilisables selon l'invention, on peut notamment citer les paraffines telles que les paraffines ayant un point de fusion entre 42 et 44°C ou entre 46 et 48°C [RN-8002-74-2], en particulier vendues par la société Merck ; les triglycérides ; les acides gras tels que l'acide dodécanoïque également appelé acide laurique dont le point de fusion est de 43,2°C ; les colophanes ; les cires (alcanes longs, i.e. comprenant au moins 12 atomes de carbone) telles que l'eicosane ou l'octadécane ; les bitumes synthétiques et les huiles végétales hydrogénées ainsi que leurs mélanges. Ces huiles peuvent être utilisées seules ou en mélanges.

Les cires sont préférées.

L'huile cristallisable a de préférence une température de fusion (T_{F}) comprise entre 30 et 60°C environ, et de préférence entre comprise entre 30 et 40°C environ.

Le matériau conforme à la présente invention est sub-micrométrique. En d'autres termes, il se présente sous la forme d'une suspension de particules sub-micrométriques solides dispersées dans une phase aqueuse ou d'une poudre de particules sub-micrométriques solides.

Par ailleurs, chacune des particules sub-micrométriques du matériau est une capsule sub-micrométrique (puisque chacune des particules sub-micrométriques comprend une enveloppe de silice et une phase grasse emprisonnée dans ladite enveloppe de silice).

Les particules sub-micrométriques dudit matériau de l'invention sont de préférence sphériques ou sensiblement sphériques.

Le diamètre des particules sub-micrométriques (ou leur plus petite dimension dans le cas où elles ne sont pas sphériques) est inférieur à 1 µm environ, de préférence varie de 400 nm à 900 nm environ, et encore plus préférentiellement de 700 à 850 nm environ.

Les particules sub-micrométriques constituant ledit matériau sont de préférence monodisperses. Elles présentent donc de préférence une distribution granulométrique des diamètres de particules étroite et en particulier, un indice de polydispersité en taille d'au plus 0,1 environ, et de préférence d'au plus 0,07 environ. La polydispersité peut être mesurée par microscopie électronique à balayage (MEB) qui fournit une déviation standard adimensionnée, et/ou par diffusion dynamique quasi-élastique de la lumière (DQEL) qui fournit un indice de polydispersité (PDI) qui est égal au carré de la déviation standard adimensionnée [J. Chem. Phys., 1972, 57, 11, 4814-4820]. La mesure de la dispersion de taille peut s'effectuer en calculant la déviation standard adimensionnée qui est le rapport de la déviation standard de la distribution de taille sur le diamètre moyen à partir d'un histogramme de taille obtenu par mesure des diamètres individuels d'une assemblée de particules sub-micrométriques (minimum 500) sur un ou plusieurs cliché MEB d'un grossissement de préférence de 8000 ou par mesure de diffusion dynamique de la lumière qui fournit le diamètre hydrodynamique moyen et le PDI d'où l'on peut déduire la déviation standard adimensionnée en prenant sa racine carrée.

Les nanoparticules superparamagnétiques présentent collectivement une aimantation nulle à champ nul et l'aimantation induite par application d'un champ magnétique est quasiment proportionnelle au champ appliqué sur toute la première partie de la courbe de leur aimantation en fonction du champ appliqué. Elles peuvent donc conduire à la formation d'une suspension dont la stabilité n'est pas perturbée par l'attraction magnétique dipolaire entre les moments magnétiques des particules nanométriques, la suspension de ces dernières ne possédant pas d'aimantation spontanée en l'absence de champ magnétique. En d'autres termes, des nanoparticules superparamagnétiques présentent le double avantage de pouvoir subir une attraction forte par un aimant ou un champ magnétique, et de ne pas s'agréger en l'absence de champ magnétique (i.e. l'absence d'aimantation spontanée en champ nul).

Dans l'invention, l'expression « nanoparticules » signifie qu'au moins 50% de la distribution en nombre desdites nanoparticules ont un diamètre inférieur à 100 nm.

Le diamètre des nanoparticules superparamagnétiques contenues dans la phase grasse varie de préférence de 10 à 20 nm environ, et encore plus préférentiellement de 12 à 16 nm environ.

Dans les gammes de diamètres précitées, une puissance spécifique de chauffe optimale dans un champ magnétique radiofréquence peut être obtenue, notamment pour les oxydes de fer superparamagnétiques.

Les nanoparticules superparamagnétiques sont de préférence homogènes en taille, et de préférence encore monodisperses.

Les nanoparticules superparamagnétiques homogènes en taille ou monodisperses présentent des propriétés de chauffe optimales par application externe d'un champ magnétique oscillant radiofréquence.

La distribution de tailles des nanoparticules superparamagnétiques est généralement mesurée par microscopie électronique à transmission (MET) et/ou par diffusion des neutrons aux petits angles (DNPA). La mesure de la dispersion de taille peut s'effectuer en calculant le rapport de la déviation standard de la distribution de taille sur le diamètre moyen à partir d'un histogramme de taille obtenu par mesure des diamètres individuels d'une assemblée de nanoparticules (minimum 500) sur un ou plusieurs cliché MET d'un grossissement de préférence de 80000 et/ou par ajustement de la courbe de l'intensité diffusée en DNPA par convolution d'un facteur de forme de particule sphérique avec une loi de distribution des diamètres.

Une homogénéité en taille peut être par exemple obtenue grâce à un procédé de tri en tailles de nanoparticules superparamagnétiques, basé sur des séparations de phase et qui sera décrit ci-après.

Dans la présente invention, l'expression « des nanoparticules homogènes en taille » signifie des nanoparticules ayant un indice de polydispersité en taille d'au plus 0,5 environ, et de préférence d'au plus 0,4 environ.

Dans la présente invention, l'expression « des nanoparticules monodisperses » signifie des nanoparticules ayant un indice de polydispersité en taille d'au plus 0,1 environ, et de préférence d'au plus 0,05 environ.

Grâce à la monodispersité ou à l'homogénéité en taille des nanoparticules superparamagnétiques, la réponse magnétique est homogène (i.e. propriétés magnétiques homogènes sur l'ensemble d'un lot de particules).

Les nanoparticules superparamagnétiques peuvent être choisies parmi les nanoparticules d'un oxyde de fer magnétique, les nanoparticules d'un oxyde mixte de fer et d'un autre métal de transition et les nanoparticules d'un oxyde ferrique de structure spinelle lacunaire de formule chimique γ-Fe₂O₃ (appelé communément maghémite).

Les nanoparticules d'un oxyde de fer magnétique peuvent être des nanoparticules de ferrite de fer, également appelée nanoparticules de magnétite, de formule Fe₃O₄.

Les nanoparticules d'un oxyde mixte de fer et d'un autre métal de transition peuvent être des nanoparticules de ferrite de formule chimique MO·Fe₂O₃ dans laquelle M désigne un métal de transition de structure spinelle différent du fer ou des nanoparticules de formule chimique M₁₋ₓM'ₓO·Fe₂O₃ dans laquelle M et M' désignent des métaux de transition différents du fer et 0 < x < 1.

M (respectivement M') peut être choisi parmi le manganèse (Mn), le zinc (Zn) et le nickel (Ni).

Selon une forme de réalisation particulièrement préférée de l'invention, les nanoparticules superparamagnétiques sont des nanoparticules de maghémite (de formule γ-Fe₂O₃).

Les nanoparticules superparamagnétiques contenues dans la phase grasse du matériau de l'invention sont fonctionnalisées avec au moins un acide gras. Cette fonctionnalisation est une fonctionnalisation par chimisorption.

Dans la présente invention, l'expression « acide gras » signifie un acide carboxylique à chaîne aliphatique, comprenant de préférence de 4 à 36 atomes de carbone, et de préférence encore de 8 à 22 atomes de carbone. L'acide gras peut être saturé ou insaturé, c'est-à-dire comportant une ou plusieurs doubles liaisons carbone-carbone. Il peut être noté Cn:m où n désigne le nombre d'atomes de carbone et m le nombre de doubles liaisons.

L'acide gras peut être choisi parmi l'acide arachidique (C20:0), l'acide stéarique (C18:0), l'acide oléique (C18:1), l'acide palmitique (C16:0), l'acide myristique (C14:0), l'acide laurique (C12:0), l'acide caprique (C10:0) et l'acide caprylique (C8:0).

Dans certains cas, l'acide gras, en particulier l'acide laurique, peut par conséquent servir à la fois d'huile cristallisable et d'agent de fonctionnalisation (i.e. stabilisant lipophile) des nanoparticules superparamagnétiques.

Les acides gras saturés tels que l'acide stéarique sont préférés, en particulier de par leur meilleure stabilité face à la dégradation par photo- ou thermo-oxydation ou par hydroperoxydation, et de par leur température de fusion plus élevée que celles des acides gras insaturés de même longueur de chaîne.

En particulier, le choix d'un acide gras dont la chaîne aliphatique est de longueur de chaîne proche ou identique à celle de l'huile cristallisable favorise la dispersion des nanoparticules superparamagnétiques dans l'huile cristallisable et donc un meilleur contrôle de la rupture de l'enveloppe de silice pour la libération d'une substance d'intérêt. Par conséquent, l'acide stéarique (C18:0) peut avantageusement être utilisé pour fonctionnaliser les nanoparticules superparamagnétiques lorsque l'huile cristallisable est une cire telle que l'eicosane. En effet, l'eicosane contient 20 atomes de carbone, sa longueur de chaîne est donc proche de celle de l'acide stéarique, tout en ayant une température de fusion (autour de 37°C) inférieure à celle de l'acide stéarique (autour de l'ordre de 50-69°C).

La fonctionnalisation permet notamment d'obtenir des nanoparticules superparamagnétiques lipophiles, chacune des nanoparticules étant revêtue de molécules d'acide gras, notamment sous la forme d'une monocouche auto-assemblée.

L'acide gras représente généralement de 10% à 30% en masse environ, et de préférence de 20% à 25% en masse environ, par rapport à la masse totale des nanoparticules superparamagnétiques fonctionnalisées.

La teneur de la phase grasse en nanoparticules superparamagnétiques fonctionnalisées est telle qu'elle permet son échauffement local à une température supérieure à sa température de fusion T_{F}. En d'autres termes, la concentration massique des nanoparticules superparamagnétiques fonctionnalisées dans la phase grasse est de préférence suffisante pour induire la transition de phase solide-liquide de l'huile cristallisable lors de l'application d'un champ magnétique alternatif radiofréquence sur les capsules sub-micrométriques, tout en garantissant une bonne stabilité colloïdale lorsque les nanoparticules superparamagnétiques sont dispersées dans la phase grasse liquide avant la préparation des capsules sub-micrométriques (e.g. étape 1) du procédé tel que décrit ci-après).

Dans un mode de réalisation particulier, les nanoparticules superparamagnétiques fonctionnalisées représentent de 0,2 à 3% en masse environ, et préférentiellement de 1 à 2,5% en masse environ, de la masse totale de la phase grasse.

Le diamètre de la phase grasse solide à la température de stockage dudit matériau varie de préférence de 400 nm à 950 nm environ, et encore plus préférentiellement de 450 à 825 nm environ.

La phase grasse du matériau conforme à l'invention peut renfermer tout type de substances d'intérêt, que celles-ci soient lipophiles ou hydrophiles. Ainsi, lorsque la ou les substances d'intérêt sont lipophiles, la phase grasse les contient sous forme solubilisée et lorsque la ou les substances d'intérêt sont hydrophiles, la phase grasse les contient sous forme dispersée (directement dans l'huile cristallisable ou dans une fraction d'eau dispersée au sein de la phase grasse (émulsion double)). Il peut également s'agir de particules solides.

Parmi les substances d'intérêt pouvant être incorporées dans la phase grasse du matériau conforme à la présente invention, on peut notamment citer les médicaments (principes actifs), les principes actifs utilisables en cosmétique, les réactifs chimiques, les colorants, les pigments, les encres telles que les encres électroniques ou magnétiques pour l'affichage ou pour les procédés de codage et d'authentification (encre infalsifiable), etc....

À titre d'exemples de médicaments, on peut mentionner les bactéricides tels que les antiseptiques et les antibiotiques, les anti-inflammatoires (Ibuprofen, Budesonide), les analgésiques, les laxatifs locaux, les hormones, les protéines, les agents anti-cancéreux (Tamoxifen, Paclitaxel), etc....

À titre d'exemples de principes actifs cosmétiques, on peut notamment citer les vitamines (e.g. rétinol), les filtres solaires, les antioxydants tels que les composés anti-radicalaires comme l'enzyme superoxyde dismutase, les parfums, les agents absorbeurs d'odeur, les agents déodorants, les agents anti transpirants, les colorants, les pigments, les émollients, les agents hydratants, etc....

À titre d'exemples de réactifs chimiques, on peut notamment citer les réactifs colorés, les indicateurs colorés tels que les indicateurs de pH, les catalyseurs, les amorceurs de polymérisation, les monomères, les complexants, etc....

La ou les substances d'intérêt représentent généralement de 0,001 à 35% en masse environ, et préférentiellement de 0,01 à 25% en masse environ de la masse totale de la phase grasse.

La phase grasse peut en outre renfermer un ou plusieurs additifs classiquement utilisés dans les émulsions et parmi lesquels on peut notamment mentionner à titre d'exemples les tensioactifs, les protecteurs ou les agents de conservation de la substance d'intérêt, tels que les antioxydants, les agents anti-UV, etc....

L'enveloppe de silice a de préférence une épaisseur et une densité suffisantes pour avoir une résistance mécanique permettant l'encapsulation de la phase grasse, tout en étant assez fine et assez peu dense pour pouvoir se rompre lors de l'application d'un champ magnétique entraînant l'échauffement local de la phase grasse constituant le cœur du matériau *via* les nanoparticules superparamagnétiques.

L'épaisseur de l'enveloppe de silice varie généralement de 30 à 50 nm environ, et préférentiellement de 36 à 46 nm environ.

La masse volumique de l'enveloppe de silice varie généralement de 1,0 à 2,5 g/cm³, et préférentiellement de 1,3 à 2,3 g/cm³ environ.

En plus de l'oxyde de silicium, l'enveloppe peut en outre comprendre un ou plusieurs oxydes métalliques de formule MeO₂ dans laquelle Me est un métal choisi parmi Zr, Ti, Th, Nb, Ta, V, W et Al. Dans ce cas, l'enveloppe est une matrice mixte de type SiO₂-MeO₂ dans laquelle la teneur massique en MeO₂ reste minoritaire par rapport à la teneur en oxyde de silicium, préférentiellement la teneur massique en MeO₂ représente de 1% à 40% environ, plus particulièrement de 5% à 30%, par rapport à la masse totale de l'enveloppe.

L'invention a pour deuxième objet un procédé de préparation d'un matériau tel que défini dans le premier objet de l'invention. Ce procédé est caractérisé en ce qu'il comporte au moins les étapes suivantes :
1) préparer une phase grasse à l'état liquide comprenant une huile cristallisable à l'état liquide ayant une température de fusion T_{F} inférieure à 100°C environ, au moins une substance d'intérêt et des nanoparticules superparamagnétiques fonctionnalisées par au moins un acide gras ;
2) mettre en contact ladite phase grasse à l'état liquide de l'étape 1) avec une phase aqueuse (PA) préalablement portée à une température T_{PA} telle que T_{PA} est supérieure à T_{F}, ladite phase aqueuse (PA) contenant des particules solides colloïdales ;
3) soumettre le mélange liquide résultant de l'étape 2) à une agitation mécanique pour obtenir une émulsion huile-dans-eau (H/E) formée de gouttelettes de phase grasse à l'état liquide dispersées dans une phase aqueuse continue et dans laquelle les particules solides colloïdales sont présentes à l'interface formée entre la phase aqueuse continue et les gouttelettes de phase grasse dispersées ;
4) laisser reposer ladite émulsion H/E puis la refroidir à une température T_{H/E} telle que T_{H/E} est inférieure à T_{F} pour provoquer la solidification de la phase grasse et obtenir une émulsion H/E formée de globules de phase grasse à l'état solide, lesdits globules étant dispersés dans la phase aqueuse continue ;
5) former une enveloppe comprenant au moins un oxyde de silicium autour de chacun desdits globules par ajout, dans la phase aqueuse continue de l'émulsion H/E de l'étape 4), et sous agitation mécanique, d'au moins un précurseur d'oxyde de silicium, d'un tensioactif TA₁ et d'une quantité suffisante d'au moins un acide pour amener la phase aqueuse à pH inférieur ou égal à 4 pour obtenir ledit matériau ;
6) éventuellement séparer ledit matériau de la phase aqueuse.

L'huile cristallisable utilisée dans l'étape 1) est telle que définie dans le premier objet de l'invention.

Les nanoparticules superparamagnétiques fonctionnalisées par au moins un acide gras et la substance d'intérêt sont telles que définies dans le premier objet de l'invention.

L'étape 1) peut être effectuée selon l'une quelconque des deux méthodes suivantes :
- porter une phase grasse comprenant une huile cristallisable solide ayant une température de fusion T_{F} inférieure à 100°C environ à une température T_{HC} telle que T_{HC} est supérieure à T_{F}, pour obtenir une phase grasse à l'état liquide ; et incorporer à la phase grasse à l'état liquide de l'étape précédente au moins une substance d'intérêt et des nanoparticules superparamagnétiques fonctionnalisées par au moins un acide gras (première méthode), ou
- mélanger une phase grasse à l'état solide comprenant une huile cristallisable solide ayant une température de fusion T_{F} inférieure à 100°C environ avec des nanoparticules superparamagnétiques fonctionnalisées par au moins un acide gras et au moins une substance d'intérêt ; et porter le mélange résultant à une température T_{HC} supérieure à T_{F}, pour obtenir une phase grasse à l'état liquide (deuxième méthode).

Les nanoparticules supermagnétiques fonctionnalisées et la substance d'intérêt peuvent être introduites (e.g. première méthode) ou mélangées (e.g. deuxième méthode) ensemble ou séparément. Le deuxième cas peut présenter un avantage dans le cas d'une substance fragile pour laquelle on doit minimiser le temps de résidence à la température T_{HC} supérieure à T_{F}.

On préfèrera avantageusement introduire la substance d'intérêt séparément, et notamment en dernier lieu dans la phase grasse à l'état liquide (première méthode).

Les particules solides colloïdales présentes dans la phase aqueuse (PA) lors de l'étape 2) peuvent être minérales ou organiques. De préférence, il s'agit de particules minérales. Les particules solides colloïdales sont de préférence des particules minérales choisies dans le groupe des oxydes, hydroxydes et sulfates de métaux, les oxydes étant particulièrement préférés. Parmi de tels oxydes, on peut tout particulièrement citer les oxydes de silicium, de titane, de zirconium ou de fer, ainsi que leurs sels tels que les silicates (par exemple les argiles). Tout autre type de particules non strictement minérales (e.g. noir de carbone, fullerène, graphène, oxyde de graphène, boronène...) peut aussi être envisagé pour stabiliser l'interface.

Afin d'être colloïdales, les particules solides présentent généralement une taille inférieure à quelques micromètres. Ainsi, les particules présentent généralement une taille moyenne comprise entre 5 et 5000 nm environ, et de préférence entre 5 et 500 nm environ.

Selon une forme de réalisation particulièrement préférée de l'invention, les particules solides colloïdales sont choisies parmi les nanoparticules d'oxyde de silicium. À titre d'exemple, on peut notamment citer les produits vendus sous la dénomination commerciale Aerosil® par la société Evonik Degussa tels que les particules solides colloïdales de silice de diamètre 7 nm vendues sous la référence Aerosil® A380.

Les nanoparticules d'oxyde de silicium présentent généralement une taille moyenne comprise entre 5 et 12 nm environ.

La quantité de particules solides colloïdales varie généralement de 0,5% à 1,7% en masse environ, et de préférence de 1,0% à 1,4% en masse environ, par rapport à la masse totale de la phase aqueuse (PA).

Selon une forme de réalisation préférée de l'invention, les particules solides colloïdales sont fonctionnalisées en surface pour les rendre plus hydrophobes. Cela permet ainsi de favoriser leur adsorption à la surface des gouttelettes de la phase grasse dispersée lors de l'étape 3) (i.e. à l'interface formée entre la phase aqueuse continue et les gouttelettes de phase grasse dispersées).

Les particules solides colloïdales peuvent ainsi être fonctionnalisées par des composés liés à leur surface par des liaisons covalentes (chimisorption) ou par adsorption de molécules d'un tensioactif TA₂ à leur surface par des liaisons électrostatiques (physisorption).

La fonctionnalisation par chimisorption peut être réalisée par traitement préalable des particules solides colloïdales, en particulier par greffage chimique d'un composé comportant des groupements hydrophobes tel qu'un trihalogénosilane ou un trialcoxysilane de formule R-Si-(OR')₃, dans laquelle R est un alkyle linéaire ou ramifié ayant de 1 à 12 atomes de carbone, en particulier ayant de 2 à 10 atomes de carbone, tout particulièrement un groupe n-octyle, portant éventuellement un groupe amino et R', identique ou différent de R, est un groupe alkyle linéaire ou ramifié ayant de 1 à 12 atomes de carbone, en particulier ayant de 1 à 6 atomes de carbone, et tout particulièrement un groupe éthyle.

À titre d'exemple de particules solides colloïdales fonctionnalisées par greffage chimique d'un composé comportant des groupements hydrophobes (e.g. composé silane), on peut notamment citer les nanoparticules de silice de 12 nm de diamètre traitées avec du dichlorodiméthyl silane, vendues sous la dénomination Aerosil®R816 par la société Evonik Degussa et les nanoparticules de silice de 16 nm de diamètre traitées avec de l'hexadécylsilane, vendues sous la dénomination Aerosil®R972 par la société Evonik Degussa.

La fonctionnalisation par physisorption permet de conférer aux particules solides colloïdales une certaine hydrophobie, l'extrémité hydrophile du tensioactif TA₂ étant adsorbée sur la surface des particules. Les tensioactifs TA₂ utilisables pour fonctionnaliser les particules sont de préférence des tensioactifs cationiques ou anioniques.

Parmi ces tensioactifs TA₂, on préfère en particulier les alkylsulfates de sodium tels que le dodécylsulfate de sodium (SDS), les sulfonates de sodium aliphatiques et/ou aromatiques tels que le dodécylbenzylsulfonate de sodium (SDBS) ou les bromures d'alkyltriméthylammonium tels que le bromure d'hexadécyltriméthyl ammonium (CTAB).

Le tensioactif TA₂ est de préférence choisi parmi les tensioactifs de charge opposée à celle de la surface des particules solides colloïdales. Ce choix permet de favoriser l'adsorption du tensioactif TA₂ à la surface des particules.

La fonctionnalisation des particules solides colloïdales par un tensioactif TA₂ peut également être réalisée *in situ*, c'est-à-dire lors de leur introduction dans la phase aqueuse (PA). Dans ce cas, la phase aqueuse (PA) renferme en outre ledit tensioactif TA₂ en une concentration préférentiellement inférieure à la concentration micellaire critique (CMC) dudit tensioactif TA₂, celui-ci venant alors s'adsorber à la surface des particules solides colloïdales lorsque celles-ci sont dans la phase aqueuse (PA). De préférence, la quantité de tensioactif TA₂ varie de 1/200 à 2/3 environ de la CMC.

Selon un mode de réalisation, le rapport massique masse de tensioactif TA₂/masse de particules solides colloïdales varie de 0,015 à 0,025 environ.

La phase aqueuse (PA) comprend principalement de l'eau (i.e. au moins 80% en volume d'eau) et éventuellement un alcool, tel que le méthanol, l'éthanol, l'isopropanol ou le butanol, et de préférence l'éthanol.

L'étape 2) est de préférence effectuée en introduisant progressivement la phase grasse issue de l'étape 1) dans la phase aqueuse (PA).

Pendant l'étape 3), l'émulsion H/E est maintenue à une température supérieure à la température T_{F}.

Avantageusement, la quantité de particules solides colloïdales présentes dans la phase aqueuse continue est ajustée en fonction de la taille moyenne en volume des gouttelettes de phase grasse à l'état liquide souhaitées dans l'émulsion H/E, telle que mesurée par diffusion dynamique quasi-élastique de la lumière laser.

En particulier, la quantité de particules solides colloïdales au sein de l'émulsion H/E varie de 35 mg à 75 mg de particules solides colloïdales/g de phase grasse, et encore plus préférentiellement de 60 mg à 68 mg de particules solides colloïdales/g de phase grasse.

Le diamètre moyen des gouttelettes de phase grasse à l'état liquide varie de préférence de 350 nm à 950 nm environ, et encore plus préférentiellement de 740 nm à 825 nm environ.

La distribution de taille des gouttelettes de la phase grasse dans l'émulsion H/E est généralement étroite (Polydispersité<20% environ).

L'agitation mécanique de l'étape 3) peut être réalisée à l'aide d'un appareil de dispersion tel que par exemple un appareil de dispersion vendu sous la dénomination commerciale Ultra-Turrax® T25 par la société Janke & Kunkel™ ou Rayneri® et/ou à l'aide d'un microfluidiseur à haute pression, tel que par exemple un microfluidiseur vendu sous la dénomination commerciale MS110 par Microfluidics™.

Selon une forme de réalisation particulièrement préférée de l'invention, l'étape 3) comprend une sous-étape d'agitation avec un appareil de dispersion conventionnel puis une sous-étape d'agitation avec un microfluidiseur.

L'utilisation d'un microfluidiseur permet notamment de diminuer la taille des gouttelettes de phase grasse à l'état liquide.

L'étape 4) est une étape de repos (sans agitation) afin d'induire un phénomène de coalescence limitée et d'obtenir une émulsion de Pickering comprenant des globules de phase grasse à l'état solide monodisperses dispersés dans la phase aqueuse continue.

L'étape précédente 3) a permis de fragmenter les gouttelettes de phase grasse à l'état liquide en une taille moyenne très inférieure à D. La quantité d'interface huile/eau est alors plus élevée que la surface susceptible d'être couverte par les particules solides colloïdales. Il existe donc initialement une fraction de surface « vierge » c'est-à-dire non protégée par les particules solides colloïdales. Ainsi, après l'arrêt de l'agitation (étape 4)), les gouttes vont coalescer et la quantité totale d'interface va diminuer. L'adsorption des particules solides colloïdales étant irréversible, la coalescence s'arrêtera lorsque les gouttelettes auront atteint un diamètre égal à D (ou supérieur à D si les particules ne forment pas une monocouche ou ne sont pas toutes adsorbées) et seront entièrement couvertes. Ce phénomène est un phénomène de « coalescence partielle » ou « coalescence limitée ». L'émulsion de Pickering est stable sur plusieurs semaines et caractérisée par une distribution étroite de la taille des globules de phase grasse à l'état solide. Les particules solides colloïdales peuvent également servir de sites de nucléation pour amorcer l'étape 5) suivante de minéralisation (procédé sol-gel) et former l'enveloppe de silice.

Lors de l'étape 5), l'ajout d'au moins un précurseur d'oxyde de silicium à pH acide provoque la condensation dudit précurseur à l'interface des globules de phase grasse à l'état solide et la formation de l'enveloppe.

Les précurseurs d'oxyde de silicium peuvent être choisis parmi les alcoxydes de silicium, et en particulier parmi le tétraméthoxyorthosilane (TMOS), le tétraéthoxyorthosilane (TEOS), le diméthyldiéthoxysilane (DMDES), le (3-mercaptopropyl)triméthoxysilane, le (3-aminopropyl)triéthoxysilane, le N-(3-triméthoxysilylpropyl)pyrrole, le 3-(2,4-dinitrophénylamino)-propyltriéthoxysilane, le N-(2-aminoéthyl)-3-aminopropyltriméthoxysilane, le phényltriéthoxysilane, le méthyltriéthoxysilane et un de leurs mélanges.

Parmi ces précurseurs, le TEOS est particulièrement préféré. Ces précurseurs peuvent être substitués, de manière totale ou partielle, par des sols de silicate.

L'épaisseur de l'enveloppe dépend de la quantité de précurseurs d'oxyde de silicium utilisée lors de l'étape 5) et du diamètre des globules de la phase grasse à l'état solide dispersés. La concentration de précurseurs d'oxyde de silicium est exprimée en moles/L (i.e. en M), par rapport à la surface totale en m² des globules de la phase grasse à l'état solide dispersés de l'émulsion H/E.

Selon une forme de réalisation préférée de l'invention, la quantité de précurseur(s) d'oxyde de silicium varie de 0,001 à 1 M/m² environ et encore plus préférentiellement de 0,005 à 0,1 M/m² environ de surface des globules de la phase grasse à l'état solide.

Pour atteindre les plus grandes épaisseurs de l'enveloppe, l'étape 5) peut être réalisée plusieurs fois jusqu'à obtenir l'épaisseur désirée.

Lorsque l'enveloppe du matériau conforme à l'invention comprend, outre l'oxyde de silicium, un oxyde métallique, on ajoute alors également à la phase aqueuse continue de l'émulsion H/E au moins un précurseur d'un oxyde métallique de formule MeO₂, ledit précurseur étant choisi parmi les alcoxydes, les chlorures et les nitrates des métaux Me choisis parmi Zr, Ti, Th, Nb, Ta, V, W et Al.

Lorsqu'ils sont utilisés, la quantité de ces précurseurs d'oxyde métallique de formule MeO₂ varie de 0,001 à 1 M/m², et préférentiellement de 0,01 à 0,6 M/m² de surface des globules de la phase grasse à l'état solide.

Le pH de la phase aqueuse lors de l'étape 5) varie de préférence de 0,01 à 4, et encore plus préférentiellement de 0,05 à 2,1.

L'acide utilisé pour ajuster le pH de la phase aqueuse peut être choisi parmi les acides minéraux et organiques parmi lesquels on peut en particulier citer l'acide chlorhydrique, l'acide acétique, l'acide nitrique, l'acide perchlorique ou l'acide sulfurique.

L'acide chlorhydrique est préféré.

Outre l'acide et le précurseur d'oxyde de silicium, un tensioactif TA₁ est également ajouté lors de l'étape 5).

Le tensioactif TA₁ est de préférence choisi parmi les tensioactifs cationiques, tels que le bromure d'hexadécyltriméthyl ammonium (CTAB).

Il permet de catalyser la réaction de condensation et de contrôler l'épaisseur de l'enveloppe des capsules sub-micrométriques constituant le matériau.

Le tensioactif TA₁ est de préférence utilisé à raison de 0,001 g à 0,1 g environ, par gramme de précurseur d'oxyde de silicium, et encore plus préférentiellement de 0,004 g à 0,05 g environ, par gramme de précurseur d'oxyde de silicium.

L'étape 5) est effectuée de préférence en ajoutant d'abord le tensioactif TA₁ puis le précurseur d'oxyde de silicium dans la phase aqueuse continue de l'émulsion.

Le précurseur d'oxyde de silicium est de préférence ajouté goutte à goutte dans la phase aqueuse continue de l'émulsion.

Lors de l'étape 6), le matériau conforme à l'invention peut être séparé de la phase aqueuse et récupéré par toute technique classique de séparation connue de l'homme du métier, telle que la filtration, la centrifugation et/ou l'utilisation de tamis. Il est ensuite de préférence lavé, par exemple à l'eau, puis séché par exemple par lyophilisation pour donner une poudre.

Le matériau obtenu à l'issue de l'étape 5) ou 6) est stable au stockage pendant plusieurs mois, à condition que la température de stockage soit inférieure à la température de fusion T_{F} de la phase grasse emprisonnée dans l'enveloppe.

Le procédé de l'invention peut comprendre en outre une étape 4') après l'étape 4) au cours de laquelle une quantité supplémentaire de particules solides colloïdales (éventuellement fonctionnalisées pour les rendre hydrophobes comme décrit ci-avant) est ajoutée. La quantité supplémentaire sert à augmenter le taux de couverture des gouttelettes pour en améliorer la stabilité. La quantité supplémentaire de particules solides colloïdales ajoutée varie de préférence de 0,006 à 0,012 g environ de particules solides colloïdales/g de phase grasse. Cette quantité supplémentaire de particules solides colloïdales éventuellement fonctionnalisées peut permettre d'empêcher l'agrégation des globules de phase grasse à l'état solide et le stockage de l'émulsion. Cela peut permettre également d'améliorer la résistance thermique de l'émulsion.

Le procédé peut comprendre en outre une étape i) préalable à l'étape 1), de préparation de nanoparticules superparamagnétiques fonctionnalisées par au moins un acide gras telles que définies dans l'invention. L'étape i) peut notamment comprendre une sous-étape i-1) de préparation des nanoparticules superparamagnétiques, et une sous-étape i-2) de fonctionnalisation desdites nanoparticules par au moins un acide gras tel que défini dans l'invention.

La sous-étape i-1) de préparation des nanoparticules superparamagnétiques peut comprendre un procédé de tri ou sélection en taille basé sur des séparations de phases successives. Ainsi, à l'issue de la sous-étape i-1) les nanoparticules superparamagnétiques peuvent être homogènes en taille.

En particulier, lorsque les nanoparticules superparamagnétiques sont des nanoparticules de maghémite, la sous-étape i-1) peut être effectuée conformément au procédé décrit par Massart et al. [IEEE Transactions on Magnetics, 1981, 17, 2, 1247-1248]. La sous-étape i-1) comprend en particulier la préparation de particules de magnétite, leur oxydation en maghémite (e.g. en présence de nitrate ferrique (FeNO₃)) et un procédé de sélection (ou tri) en taille. Le procédé de sélection (ou tri) en taille peut être en particulier effectué selon la procédure décrite par Massart et al. [Journal of Magnetism and Magnetic Materials, 1995, 149, 1-2, 6-7]. Les nanoparticules de maghémite sont alors homogènes en taille (i.e. distribution de taille réduite).

Le matériau conforme à l'invention peut être utilisé sous forme de poudre ou de dispersion dans un solvant pour délivrer la ou les substances d'intérêt présente(s) dans la phase grasse solide emprisonnée dans l'enveloppe à base d'oxyde de silicium.

L'invention a donc également pour objet l'utilisation d'un matériau conforme à l'invention et tel que décrit précédemment pour la délivrance magnéto-stimulée d'au moins une substance d'intérêt.

Le matériau conforme à l'invention est par conséquent destiné à être utilisé pour la délivrance magnéto-stimulée d'au moins une substance d'intérêt.

La délivrance de la substance d'intérêt est obtenue par rupture de l'enveloppe sous l'effet d'un champ magnétique alternatif radiofréquence, entraînant une élévation locale (i.e. au niveau du cœur de la capsule) de la température à une température de délivrance T_{D} telle que T_{D} > T_{F}.

La fréquence du champ magnétique alternatif radiofréquence peut être comprise entre 3 kHz et 30 MHz environ (champ basse-fréquence), entre 30 et 300 MHz environ (champ haute fréquence ou VHF) ou entre 300 MHz et 3 GHz environ (champ ultra-haute fréquence ou UHF). Pour des applications *in vivo*, la fréquence du champ est de préférence comprise entre 30 et 900 kHz environ.

L'intensité du champ magnétique alternatif radiofréquence peut varier de 2 à 40 kA/m environ (i.e. une induction magnétique B comprise entre 2,5 et 50 mT).

Selon un mode de réalisation avantageux, notamment pour des applications *in vivo*, le produit de la fréquence par l'intensité du champ alternatif radiofréquence est d'au plus 5x10⁹ A/m/s.

Le champ magnétique alternatif radiofréquence peut être appliqué par tout moyen approprié connu de l'homme du métier, notamment en utilisant un appareil d'imagerie par résonance magnétique (IRM).

A titre d'exemple, et lorsque la substance d'intérêt est un médicament, l'huile cristallisable présente dans la phase grasse est de préférence choisie parmi les huiles cristallisables ayant un point de fusion supérieur à 36°C environ. Ainsi, lorsque ledit matériau est incorporé dans une composition pharmaceutique et que cette composition est administrée à un patient, par exemple par voie orale, la composition ingérée va se trouver à la température du corps, en général 37°C. Afin de provoquer la rupture de la capsule, le corps entier du patient, ou bien juste une partie, pourra être plongé dans un champ magnétique de fréquence et d'amplitude convenablement choisis, entraînant le chauffage microscopique/local de la phase grasse et son expansion volumique, et ainsi la rupture de l'enveloppe de silice et la délivrance du médicament.

Selon un autre exemple, la substance d'intérêt est un principe actif cosmétique et le matériau fait partie des composants d'une composition cosmétique à application topique, telle qu'une poudre, une crème ou un gel. L'application d'un champ magnétique de fréquence et d'amplitude convenablement choisis peut induire progressivement l'échauffement local de la phase grasse du matériau à une température supérieure à T_{F} (sans brûler la peau) et permettre une incorporation lente et contrôlée de la substance d'intérêt dans les pores de la peau, notamment en évitant toute explosion des capsules.

Si la composition cosmétique se présente sous la forme d'une poudre, l'application d'un champ magnétique de fréquence et d'amplitude convenablement choisis peut s'accompagner d'un changement de texture (transformation de la poudre en une composition ayant un toucher gras dû à la rupture de l'enveloppe) en évitant toute projection de poudre dans les yeux ou les zones sensibles du corps humain.

A titre d'autres exemples d'utilisation du matériau conforme à l'invention, on peut notamment citer l'utilisation dudit matériau dans le domaine de l'imagerie médicale, par exemple comme agent de contraste pour l'imagerie par résonance magnétique (IRM). En effet, les nanoparticules superparamagnétiques encapsulées peuvent apporter des propriétés de contraste des images IRM en modifiant les temps de relaxation des noyaux hydrogènes de l'eau et des graisses dans les organes. Une fois dans l'organisme, le matériau peut en sus être guidé vers un organe ou en particulier une tumeur grâce à l'application d'un champ magnétique statique et inhomogène (gradient de champ), ce qui est un autre avantage du contrôle magnétique à distance des capsules (en plus de la libération d'actif magnéto-induit). On peut donc utiliser ledit matériau pour le guidage magnétique vers un organe ou une tumeur ciblé(e).

L'invention a également pour objet l'utilisation du matériau tel que décrit ci-dessus, à titre d'ingrédient, pour la préparation de produits pharmaceutiques, cosmétiques ou alimentaires, ainsi que les produits pharmaceutiques, cosmétiques ou alimentaires, renfermant, à titre d'ingrédient, au moins un matériau conforme à l'invention.

Ces compositions peuvent renfermer les supports pharmaceutiques, cosmétiques ou alimentaires classiques et bien connus de l'homme du métier, ainsi qu'un ou plusieurs tensioactifs destinés à favoriser la libération de la phase grasse liquide lors de la rupture de la capsule.

La présente invention est illustrée par les exemples de réalisation suivants, auxquels elle n'est cependant pas limitée.

### EXEMPLES

Les matières premières utilisées dans les exemples qui suivent sont listées ci-après :
- Bromure de cétyl-triméthylammonium (CTAB), pureté ≥ 98%, société Sigma-Aldrich ;
- éther diéthylique, société Sigma-Aldrich ;
- solution d'acide chlorhydrique à 37% en masse, société Sigma-Aldrich ;
- méthanol technique, société Sigma-Aldrich ;
- solution d'acide nitrique à 69% en masse, société Sigma-Aldrich ;
- acétone technique, société Sigma-Aldrich ;
- solution d'hydroxyde d'ammonium à 30% en masse, société Sigma-Aldrich ;
- nitrate ferrique non hydraté, pureté ≥ 98%, société Alfa Aesar ;
- solution de chlorure ferrique à 45% en masse, société Sigma-Aldrich ;
- chlorure ferreux tétra hydraté, pureté ≥ 98%, société Alfa Aesar ;
- acide oléique, pureté 90%, société Sigma-Aldrich ;
- acide stéarique, pureté 95%, société Sigma-Aldrich ;
- chloroforme, société Sigma-Aldrich ;
- nanoparticules de silice de 7 nm de diamètre, vendues sous la dénomination Aerosil™ A380 par la société Evonik Degussa ;
- n-eicosane (C₂₀H₄₂), pureté 99%, point de fusion = 36°C, société Aldrich ; et
- Tetraéthoxyorthosilane (TEOS), société Sigma-Aldrich.

Ces matières premières ont été utilisées telles que reçues des fabricants, sans purification supplémentaire.

Les matériaux obtenus ont été caractérisés par plusieurs techniques décrites ci-dessous.

Afin de déterminer la concentration en acide gras des nanoparticules d'oxyde de fer fonctionnalisées, des analyses thermogravimétriques (ATG) ont été effectuées à l'aide d'une balance thermique vendue sous la dénomination commerciale Setaram Instrumentation™ en utilisant un flux d'air et en chauffant l'échantillon de 20 à 800°C avec une montée en température de 10°C par minute.

Les matériaux ont été observés à l'aide d'un microscope électronique à balayage (MEB) vendu sous la référence TM-1000 par la société Hitachi. Le matériau analysé a été préalablement soit séché à température ambiante, soit lyophilisé pendant 12 h à -80°C à l'aide d'un appareil à lyophiliser vendu sous la dénomination Alpha 2-4 LD Plus par la société Christ. Tous les matériaux ont été recouverts d'or avant d'être observés en MEB.

Les expériences d'hyperthermie magnétique ont été effectuées à l'aide d'un appareil de brasage tendre à induction vendu sous la dénomination commerciale Minimax Junior™ 1TS de la société italienne Seit Elettronica revendu par la société Maxmatic. L'appareil utilisé comprend un générateur de 3,5 kW à transistor MOSFET (acronyme anglais de *Métal Oxide Semiconductor Field Effect Transistor* - qui se traduit par *transistor à effet de champ à structure oxyde de métal semiconducteur ou transistor à effet de champ à grille isolée)* produisant un champ magnétique alternatif quasi-sinusoïdal à une radiofréquence de 755 kHz dans un circuit résonant comprenant une bobine d'induction 4 tours (diamètre interne de 50 mm et hauteur de 32 mm) réfrigéré par circulation interne d'eau froide (i.e. à l'intérieur des fils conducteurs). L'intensité du champ magnétique alternatif a été estimée à 10,2 kA/m à puissance totale (747 V, 234 Amps) grâce à un logiciel de simulation par éléments finis des problèmes de magnétisme FEMM (acronyme anglais de *Finite Element Model Magnetics* - qui se traduit par *modélisation par éléments finis en magnétisme*) (http://www.femm.info/).

### Exemple 1 : Préparation et caractérisations de matériaux conformes à l'invention

### 1) Préparation de matériaux conformes à l'invention

### 1.1) Préparation des nanoparticules superparamagnétiques monodisperses fonctionnalisées par un acide gras

Des nanoparticules superparamagnétiques de maghémite de formule γ-Fe₂O₃ ont été préparées selon le procédé décrit par Massart et al. [IEEE Transactions on Magnetics, 1981, 17, 2, 1247-1248].

Dans un premier temps, des nanocristaux polydisperses de magnétite de formule Fe₃O₄ (ou FeO·Fe₂O₃) ont été préparés en phase aqueuse par coprécipitation alcaline. Pour ce faire, 180 g d'un chlorure ferreux et 367 ml d'une solution de chlorure ferrique à 45% (i.e. 4,1 M) ont été introduits selon les proportions molaires non stœchiométriques 0,9:1,5 dans une solution comprenant 100 ml d'HCl concentré à 37% (environ 12,2 M) dilués dans 500 ml d'eau. La solution résultante a ensuite été diluée avec de l'eau pour former un volume total de solution aqueuse de 3 litres. La solution aqueuse résultante a été placée sous agitation mécanique vigoureuse (environ 800 tours par minute) et 1 litre d'une solution d'ammoniaque concentrée à 30% a été ajoutée le plus rapidement possible afin de permettre la coprécipitation. Un précipité noir caractéristique de la magnétite a ainsi été obtenu. La suspension résultante a été agitée pendant 30 minutes et décantée à l'aide d'un aimant permanent en ferrite vendu sous la dénomination commerciale Calamit Magneti™ de dimensions 152x101x25,4 mm³, jusqu'à ce que le surnageant soit incolore (au moins 10 minutes). L'aimant a été utilisé pour accélérer l'extraction du surnageant puis son aspiration à l'aide d'une fiole à vide. Après le lavage du précipité avec 1 litre d'eau distillée puis à nouveau la sédimentation magnétique, le floculat a été acidifié avec une solution comprenant 360 ml d'acide nitrique à 69% (15 M) dilués dans 1,6 litres d'eau distillée. Après 30 minutes d'agitation, la suspension désormais acide a été à nouveau décantée sur l'aimant permanent jusqu'à obtenir un surnageant limpide, qui a été ensuite aspiré puis éliminé.

Dans un deuxième temps, l'intégralité des nanocristaux polydisperses de magnétite Fe₃O₄ colloïdale obtenus précédemment a été oxydé en maghémite de formule γ-Fe₂O₃ par ajout d'une solution comprenant 323 g de nitrate ferrique (FeNO₃) dilués dans 800 mL d'eau portée à ébullition (90-100°C) sous agitation mécanique. La suspension résultante est devenue rouge brique, couleur caractéristique de la maghémite. Le précipité a été décanté sur l'aimant permanent, le surnageant aspiré, puis 360 ml d'acide nitrique à 69% dilués dans 1,6 litre d'eau distillée lui ont été ajoutés. Afin de retirer tous les ions de nitrate ferrique en excès, la suspension a été lavée une fois avec 1 litre d'acétone (agitation 10 minutes, décantation magnétique puis aspiration du surnageant), puis deux fois avec 500 ml d'éther diéthylique (agitation 10 minutes, décantation magnétique puis aspiration du surnageant). Enfin, les solvants organiques ont été évaporés par agitation mécanique sous une hotte aspirante, les particules de maghémite ayant été au préalable redispersées dans de l'eau acidifiée à un pH de 2 environ par ajout d'acide nitrique, pour former une suspension de nanoparticules de maghémite stable mais polydisperses, avec des tailles allant de 5 nm à 20 nm environ.

Dans un troisième temps, les nanoparticules de maghémite obtenues ont subi un procédé de sélection (ou tri) en taille tel que décrit par Massart et al. [Journal of Magnetism and Magnetic Materials, 1995, 149, 1-2, 6-7]. Ce procédé a permis de réduire la polydispersité de nanoparticules de maghémite. Ce procédé de tri granulométrique est bien connu de l'homme du métier. Il est basé sur la séparation de phase par fractionnement. Pour ce faire, une solution d'acide nitrique en excès (HNO₃ à 15 M) a été ajoutée à la suspension mère de nanoparticules de maghémite polydisperses telle que préparée ci-dessus. Cela a permis ainsi de diminuer le pH (initialement à 2) jusqu'à 0,8 et d'induire une augmentation de la force ionique et ainsi la formation d'une phase supérieure, le surnageant, plus diluée en fraction solide et contenant des nanoparticules de plus petites tailles, et d'une phase inférieure plus concentrée, attirée par l'aimant, contenant des particules de plus grandes tailles. Les deux phases ont ensuite été séparées après décantation à l'aide d'un aimant tel que décrit ci-dessus et aspiration de la phase supérieure. En répétant ces étapes sur chacune des fractions, il a été possible d'obtenir une fraction comprenant des nanoparticules triées de maghémite de taille comprise entre 12 et 15 nm environ.

Les nanoparticules telles que préparées ci-dessus ont ensuite été fonctionnalisées soit par de l'acide stéarique, soit par de l'acide oléique.

En ce qui concerne les nanoparticules fonctionnalisées avec de l'acide oléique, un mélange comprenant les proportions molaires suivantes en acide oléique/ammoniaque/fer de 1/1/5, a été chauffé à 60°C environ pendant 30 minutes sous agitation mécanique. Le mélange résultant s'est séparé entre une phase aqueuse moussante et une pâte hydrophobe de couleur marron-noir, qui a été sédimentée sur l'aimant permanent après refroidissement à température ambiante (20°C environ), puis lavée trois fois avec du méthanol et séchée sous vide pendant 30 min pour retirer le plus possible d'eau.

En ce qui concerne les nanoparticules fonctionnalisées avec de l'acide stéarique, un mélange comprenant les proportions molaires en acide stéarique/ammoniaque/fer de 1/1/5, a été chauffé à 70°C environ pendant 30 minutes sous agitation mécanique. Le mélange résultant s'est séparé entre une phase aqueuse moussante et une pâte hydrophobe de couleur marron-noir, qui a été sédimentée sur l'aimant permanent après refroidissement à température ambiante (20°C environ), puis lavée trois fois avec du méthanol et séchée sous vide pendant 30 min pour retirer le plus possible d'eau.

La fonctionnalisation a conféré aux nanoparticules de maghémite un caractère lipophile, permettant ainsi de les incorporer dans une phase grasse telle que définie dans l'invention. Ainsi, les nanoparticules de maghémite triées en taille fonctionnalisées vont former une suspension stable dans l'huile cristallisable lorsque celle-ci est sous la forme liquide et demeurent au cœur des capsules sub-micrométriques au cours de leur fabrication.

La puissance spécifique massique de chauffe ou SAR (acronyme anglais de *Specific Absorption Rate* - qui se traduit littéralement par taux d'absorption spécifique et s'exprime en Watts par gramme) des nanoparticules superparamagnétiques de maghémite **fonctionnalisées** a été mesurée à 280 W/g environ dans l'eau dans les conditions de champ magnétique alternatif employées (10,2 kA/m à 755 kHz). Cette puissance diminue dans l'eicosane à 8 W/g environ (nanoparticules de maghémite fonctionnalisées par l'acide oléique) ou à 6 W/g (nanoparticules de maghémite fonctionnalisées par l'acide stéarique) vraisemblablement du fait de l'immobilisation des nanoparticules magnétiques dans la cire, ce qui réduit fortement la puissance thermique dissipée par l'oscillation des moments magnétiques (phénomène amplifié avec l'acide stéarique qui est aussi cristallisé à température ambiante).

Des analyses par thermogravimétrie (ATG) ont montré que les nanoparticules de maghémite fonctionnalisées avec de l'acide oléique comprennent environ 130 mg d'acide oléique par gramme de pâte solide de nanoparticules de maghémite fonctionnalisées avec l'acide oléique et les nanoparticules fonctionnalisées avec de l'acide stéarique comprennent environ 220 mg d'acide stéarique par gramme de pâte solide de nanoparticules de maghémite fonctionnalisées avec l'acide stéarique.

### 1.2) Préparation de particules solides colloïdales fonctionnalisées

1,18 g de nanoparticules de silice Aerosil™ A380 ont été dispersés dans 100 mL d'eau distillée, à l'aide d'une cuve à ultrasons. On a ensuite ajouté à cette dispersion, 22,4 mg de CTAB, cette quantité représentant environ un facteur 0,65 de la concentration micellaire critique du CTAB (CMC = 0,9.10⁻³ mol/l). La surface des nanoparticules de silice étant chargée négativement, le CTAB (tensioactif cationique) vient s'adsorber à la surface des particules de silice et permet ainsi de leur conférer un caractère hydrophobe. Ce caractère hydrophobe leur permet de stabiliser l'interface phase grasse-phase aqueuse continue de l'émulsion lors de sa préparation. On a obtenu une dispersion de nanoparticules de silice fonctionnalisées en surface dans une phase aqueuse. Le rapport massique masse tensioactif/masse particules solides colloïdales était de 0,019 environ.

### 1.3) Préparation des émulsions

Afin de préparer deux émulsions, dont les compositions sont précisées dans le tableau 1 ci-après, une quantité donnée de pâte solide de nanoparticules de maghémite fonctionnalisées par l'acide oléique ou par l'acide stéarique telles que préparées dans l'exemple 1.1) a été ajoutée à 18 g d'eicosane (huile cristallisable), afin d'obtenir une suspension comprenant une concentration finale en oxyde de fer de 12 g/L environ. La phase grasse résultante a été chauffée à 55°C environ afin de faire fondre l'eicosane [étape 1)] dans lequel les nanoparticules supermagnétiques triées en taille fonctionnalisées forment une suspension homogène et limpide.

Des analyses par diffusion dynamique de la lumière ont permis de montrer que les nanoparticules de maghémite fonctionnalisées par de l'acide oléique en suspension dans la phase grasse présentent une taille hydrodynamique moyenne de 25 nm environ avec un indice de polydispersité (PDI) de 0,36 environ (mesure effectuée par DQEL sur une suspension à 4 g/L) et les nanoparticules de maghémite fonctionnalisées par de l'acide stéarique en suspension dans la phase grasse présentent une taille hydrodynamique moyenne de 24 nm environ avec un indice de polydispersité (PDI) de 0,4 (mesure effectuée par DQEL sur une suspension à 2 g/L) environ. Cela montre que les nanoparticules de maghémite sont individuellement dispersées (i.e. pas de formation d'agrégats ni de clusters) et revêtues d'une monocouche auto-assemblée de molécules d'acides gras assurant une répulsion stérique efficace contre les forces attractives de Van der Waals et dipolaires magnétiques entre les grains.

En parallèle, la phase aqueuse comprenant des nanoparticules de silice fonctionnalisées par du CTAB en suspension telle que préparée dans l'exemple 1.2) a été chauffée à 55°C environ. Puis, une quantité donnée de phase grasse telle que préparée ci-dessus a été incorporée progressivement à une quantité donnée de phase aqueuse précitée [étape 2)] et l'ensemble a été agité vigoureusement et homogénéisé à l'aide d'un agitateur vendu sous la dénomination Ultra-Turrax™ T25 par la société Janke & Kunkel™ équipé d'un outil de dispersion S25 N-25F, à une vitesse de 20000 tours environ pendant 1 min. Afin d'obtenir des gouttelettes de phase grasse plus petites, le mélange résultant a été transféré dans un microfluidiseur à haute pression vendu sous la dénomination commerciale MS110 par Microfluidics™ et microfluidisé pendant 30 secondes environ à une pression de 95 MPa environ. Pendant la préparation de l'émulsion, celle-ci a été maintenue à 55°C, afin d'éviter toute cristallisation de l'huile cristallisable [étape 3)].

**Tableau 1**

| **Émulsion H/E** | **Quantité de nanoparticules de maghémite fonctionnalisées (en g)** | **Quantité de phase grasse dans l'émulsion (en g)** | **Quantité de phase aqueuse dans l'émulsion (en g)** | **Quantité de nanoparticules de silice par gramme de phase grasse dans l'émulsion (en mg)** |
|---|---|---|---|---|
| **E-OA** | 0,31 | 18,31 | 101,2024 | 64 |
| **E-SA** | 0,33 | 18,33 | 101,2024 | 64 |

Le diamètre moyen des gouttelettes de phase grasse à l'état liquide était de 740 nm environ pour l'émulsion **E-OA** et 900 nm environ pour l'émulsion **E-SA**.

Ensuite, l'émulsion résultante a été laissée au repos dans un four à 55°C pendant 10 min afin de faire apparaître le phénomène de coalescence limitée. Une fois refroidie à une température inférieure au point de fusion de l'huile cristallisable (eicosane) [étape 4)], une petite quantité de nanoparticules de silice fonctionnalisées avec du CTAB (solution de 0,17 g de nanoparticules fonctionnalisées avec du CTAB dispersées dans 4,8 ml d'eau) a été ajoutée dans l'émulsion **E-OA** [étape 4')]. L'addition de cette quantité supplémentaire de particules solides colloïdales permet d'empêcher l'agrégation des particules de cire et le stockage de l'émulsion à température ambiante. 119,5 g environ de chacune des émulsions **E-OA** et **E-SA** comprenant des globules de phase grasse à l'état solide dispersés dans une phase aqueuse continue ont ainsi été obtenus.

### 1.4) Préparation des capsules sub-micrométriques conformes à l'invention : formation de l'enveloppe de silice (étape de minéralisation)

Dans cette étape [étape 5)], on a réalisé la formation de l'enveloppe de silice autour des globules de phase grasse à l'état solide.

Les deux émulsions **E-OA** et **E-SA** ont été diluées de 18% massique à 2% massique et le pH des émulsions a été ajusté à 0,2 environ, c'est-à-dire à une valeur inférieure au point isoélectrique de la silice, par ajout à la fois de 7 g d'une solution d'acide chlorhydrique à 37% en masse (environ 12,2 M) et de 80 g d'une solution aqueuse contenant 0,21 g de CTAB.

5 g de TEOS a alors été ajouté goutte à goutte aux deux émulsions pour atteindre la quantité notée dans le tableau 2 ci-dessous. Pendant l'addition, la solution a été placée sous agitation magnétique à une vitesse de 450 tours/min, cette vitesse ne modifiant pas la distribution de la taille des gouttes. La dispersion résultante a été placée toute la nuit dans des tubes à essais de 50 mL sous agitation continue sur roue à 25 tours/min dans une chambre thermostatée à 20°C pour laisser l'enveloppe de silice se former (minéralisation).

A la fin de la minéralisation, des capsules sub-micrométriques de silice ont été récupérées après plusieurs cycles de centrifugation-redispersion plusieurs fois dans de l'eau distillée [étape 6)]. Le matériau obtenu a été conservé dans l'eau pure pendant plusieurs mois. Aucune altération des capsules sub-micrométriques n'a été observée pendant cette période.

**Tableau 2**

| **Émulsion** | **Quantité de CTAB par g de TEOS (en g)** | **Quantité de TEOS (en M/m²)** |
|---|---|---|
| **E-OA** | 0,042 | 0,039 |
| **E-SA** | 0,042 | 0,043 |

### 2) Résultats des caractérisations

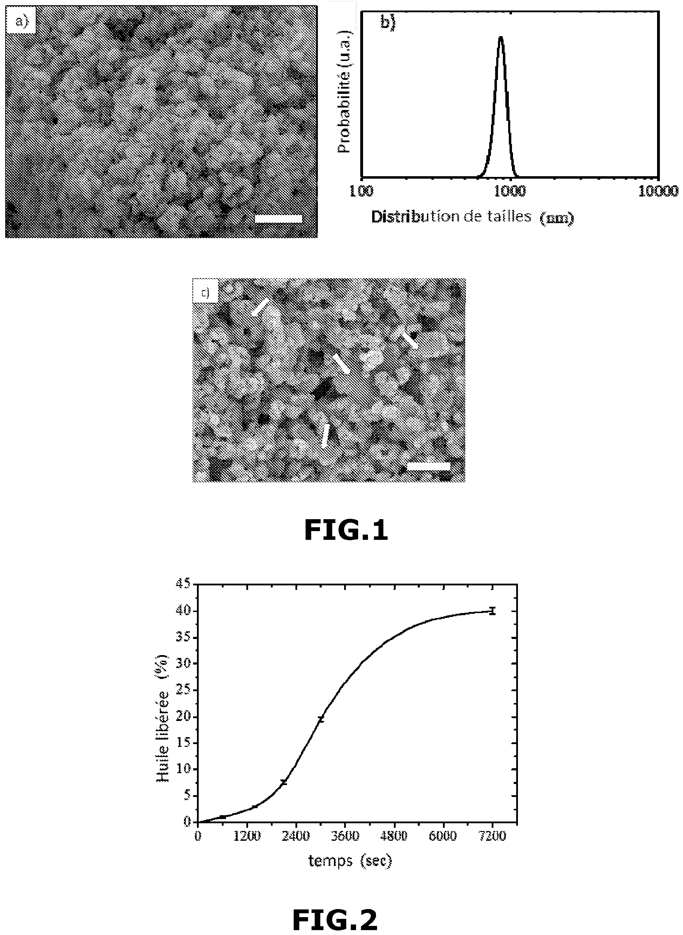

La figure 1 annexée représente une photographie MEB prise lors de l'observation d'un matériau conforme à l'invention obtenu par minéralisation de l'émulsion **E-SA** (figure la), puis sa distribution en taille montrant un matériau comprenant des particules ayant une taille sub-micrométrique centrée autour de 825 nm (figure 1b), et enfin une photographie MEB prise lors de l'observation d'un matériau conforme à l'invention obtenu par minéralisation de l'émulsion **E-SA** après rupture de l'enveloppe par application d'un champ magnétique alternatif radiofréquence (figure 1c). Sur les figures la et 1c, la barre d'échelle représente 5 µm et sur la figure 1c, la flèche blanche pointe sur la zone de fracture provoquée par l'expansion de la phase grasse.

### Exemple 2 : Profil de libération des matériaux conformes à l'invention

Dans cet exemple, on illustre la rupture d'un matériau conforme à l'invention obtenu par minéralisation de l'émulsion **E-SA**.

Le matériau a été exposé à un champ magnétique alternatif à une radiofréquence de 755 kHz environ et une intensité de 10,2 kA/m environ pendant un temps variable : 600, 1380, 2100, 3000 et 7200 secondes.

La figure 2 montre la quantité d'huile cristallisable libérée (en pourcentage) en fonction du temps d'application du champ magnétique alternatif (en secondes).

D'après la figure 2, il apparaît que 20% environ de l'huile est libérée après 50 minutes (3000 s) d'application d'un champ magnétique alternatif radiofréquence de 10,2 kA/m à 755 kHz, jusqu'à atteindre 40% après 2 heures (7200 s) d'application d'un champ magnétique alternatif radiofréquence de 10,2 kA/m à 755 kHz.

Il est possible d'accélérer la vitesse de libération de l'huile et donc celle d'une substance d'intérêt en augmentant la quantité de nanoparticules superparamagnétiques dans le cœur des capsules (modification de l'acide gras par exemple) et/ou leur puissance spécifique massique de chauffe par une modification de la forme et du type de nanoparticules superparamagnétiques employées, par exemple avec des « nanocubes » ou des « nano-fleurs », c'est à dire des nanoparticules multi-cœurs, qui peuvent atteindre des valeurs de SAR de plus de 1000 W/g.

## Revendications

1. Matériau sous la forme de particules solides contenant une phase grasse solide à la température de stockage dudit matériau et une enveloppe continue comprenant au moins un oxyde de silicium et emprisonnant ladite phase grasse, ladite phase grasse comprenant :
- une huile cristallisable ayant une température de fusion (T_{F}) inférieure à 100°C, et étant choisie parmi les matières grasses et les mélanges de matières grasses, d'origine naturelle ou synthétique, dont le point de fusion est supérieur à 15°C, et
- au moins une substance d'intérêt,
ladite température de stockage étant toujours inférieure au point de fusion de ladite huile cristallisable, et
ledit matériau étant **caractérisé en ce qu'**il est sub-micrométrique et **en ce que** la phase grasse comprend en outre des nanoparticules superparamagnétiques fonctionnalisées en surface par au moins un acide gras.

2. Matériau selon la revendication 1, **caractérisé en ce que** l'huile cristallisable est choisie parmi les paraffines, les triglycérides, les acides gras, les colophanes, les cires, les huiles végétales hydrogénées ainsi que leurs mélanges, et les bitumes synthétiques.

3. Matériau selon la revendication 1 ou 2, **caractérisé en ce que** le diamètre des particules varie de 400 nm à 900 nm.

4. Matériau selon l'une quelconque des revendications précédentes, **caractérisé en ce que** les particules constituant ledit matériau sont monodisperses.

5. Matériau selon l'une quelconque des revendications précédentes, **caractérisé en ce que** le diamètre des nanoparticules superparamagnétiques contenues dans la phase grasse varie de 10 à 20 nm.

6. Matériau selon l'une quelconque des revendications précédentes, **caractérisé en ce que** les nanoparticules superparamagnétiques sont des nanoparticules de maghémite.

7. Matériau selon l'une quelconque des revendications précédentes, **caractérisé en ce que** l'acide gras est choisi parmi l'acide arachidique, l'acide stéarique, l'acide oléique, l'acide palmitique, l'acide myristique, l'acide laurique, l'acide caprique et l'acide caprylique.

8. Matériau selon l'une quelconque des revendications précédentes, **caractérisé en ce que** les nanoparticules superparamagnétiques fonctionnalisées représentent de 0,2 à 3% en masse, de la masse totale de la phase grasse.

9. Matériau selon l'une quelconque des revendications précédentes, **caractérisé en ce que** l'épaisseur de l'enveloppe de silice varie de 30 à 50 nm.

10. Procédé de préparation d'un matériau tel que défini à l'une quelconque des revendications précédentes, **caractérisé en ce qu'**il comporte au moins les étapes suivantes :
1) préparer une phase grasse à l'état liquide comprenant une huile cristallisable à l'état liquide ayant une température de fusion T_{F} inférieure à 100°C, et étant choisie parmi les matières grasses et les mélanges de matières grasses d'origine naturelle ou synthétique dont le point de fusion est supérieur à 15 °C, au moins une substance d'intérêt et des nanoparticules superparamagnétiques fonctionnalisées par au moins un acide gras ;
2) mettre en contact ladite phase grasse à l'état liquide de l'étape 1) avec une phase aqueuse (PA) préalablement portée à une température T_{PA} telle que T_{PA} est supérieure à T_{F}, ladite phase aqueuse (PA) contenant des particules solides colloïdales ;
3) soumettre le mélange liquide résultant de l'étape 2) à une agitation mécanique pour obtenir une émulsion huile-dans-eau (H/E) formée de gouttelettes de phase grasse à l'état liquide dispersées dans une phase aqueuse continue et dans laquelle les particules solides colloïdales sont présentes à l'interface formée entre la phase aqueuse continue et les gouttelettes de phase grasse dispersées ;
4) laisser reposer ladite émulsion H/E puis la refroidir à une température T_{H/E} telle que T_{H/E} est inférieure à T_{F} pour provoquer la solidification de la phase grasse et obtenir une émulsion H/E formée de globules de phase grasse à l'état solide, lesdits globules étant dispersés dans la phase aqueuse continue ;
5) former une enveloppe comprenant au moins un oxyde de silicium autour de chacun desdits globules par ajout, dans la phase aqueuse continue de l'émulsion H/E de l'étape 4), et sous agitation mécanique, d'au moins un précurseur d'oxyde de silicium, d'un tensioactif TA₁ et d'une quantité suffisante d'au moins un acide pour amener la phase aqueuse à pH inférieur ou égal à 4 pour obtenir ledit matériau ;
6) éventuellement séparer ledit matériau de la phase aqueuse.

11. Procédé selon la revendication 10, **caractérisé en ce que** les particules solides colloïdales sont choisies parmi les nanoparticules d'oxyde de silicium.

12. Procédé selon la revendication 10 ou 11, **caractérisé en ce que** les particules solides colloïdales sont fonctionnalisées en surface pour les rendre plus hydrophobes par adsorption de molécules d'un tensioactif TA₂ à leur surface par des liaisons électrostatiques.

13. Procédé selon l'une quelconque des revendications 10 à 12, **caractérisé en ce que** l'agitation mécanique de l'étape 3) est réalisée à l'aide d'un appareil de dispersion et/ou à l'aide d'un microfluidiseur à haute pression.

14. Matériau tel que défini à l'une quelconque des revendications 1 à 9 ou obtenu selon le procédé tel que défini à l'une quelconque des revendications 10 à 13, pour son utilisation pour la délivrance magnéto-stimulée d'au moins une substance d'intérêt.

15. Utilisation d'un matériau tel que défini à l'une quelconque des revendications 1 à 9 ou obtenu selon le procédé tel que défini à l'une quelconque des revendications 10 à 13, dans le domaine de l'imagerie médicale, notamment comme agent de contraste ou pour le guidage magnétique vers un organe ou une tumeur ciblé(e).

## Patentansprüche

1. Material in Form fester Partikel, enthaltend eine feste Fettphase bei der Lagertemperatur des Materials und eine kontinuierliche Hülle, die mindestens ein Siliciumoxid umfasst und die Fettphase einschließt, wobei die Fettphase umfasst:
- ein kristallisierbares Öl mit einer Schmelztemperatur (T_{F}) unter 100 °C, und ausgewählt aus den Fetten und den Fettgemischen natürlichen oder synthetischen Ursprungs mit einem Schmelzpunkt über 15 °C, und
- mindestens eine Substanz von Interesse,
wobei die Lagertemperatur immer unter dem Schmelzpunkt des kristallisierbaren Öls liegt, und
das Material **dadurch gekennzeichnet ist, dass** es submikrometrisch ist und dass die Fettphase ferner superparamagnetische Nanopartikel umfasst, die auf der Oberfläche durch mindestens eine Fettsäure funktionalisiert sind.

2. Material nach Anspruch 1, **dadurch gekennzeichnet, dass** das kristallisierbare Öl aus den Paraffinen, den Triglyceriden, den Fettsäuren, den Kolophonium, den Wachsen, den hydrierten Pflanzenölen sowie ihren Gemischen und den synthetischen Bitumen ausgewählt ist.

3. Material nach Anspruch 1 oder 2, **dadurch gekennzeichnet, dass** der Durchmesser der Partikel von 400 nm bis 900 nm schwankt.

4. Material nach einem der vorangehenden Ansprüche, **dadurch gekennzeichnet, dass** die Partikel, die das Material bilden, monodispergiert sind.

5. Material nach einem der vorangehenden Ansprüche, **dadurch gekennzeichnet, dass** der Durchmesser der superparamagnetischen Nanopartikel, die in der Fettphase enthalten sind, von 10 bis 20 nm schwankt.

6. Material nach einem der vorangehenden Ansprüche, **dadurch gekennzeichnet, dass** die superparamagnetischen Nanopartikel Maghemit-Nanopartikel sind.

7. Material nach einem der vorangehenden Ansprüche, **dadurch gekennzeichnet, dass** die Fettsäure aus der Arachinsäure, der Stearinsäure, der Oleinsäure, der Palmitinsäure, der Myristinsäure, der Laurinsäure, der Caprinsäure und der Caprylsäure ausgewählt ist.

8. Material nach einem der vorangehenden Ansprüche, **dadurch gekennzeichnet, dass** die funktionalisierten superparamagnetischen Nanopartikel 0,2 bis 3 Ma% der Gesamtmasse der Fettphase darstellen.

9. Material nach einem der vorangehenden Ansprüche, **dadurch gekennzeichnet, dass** die Dicke der Siliciumdioxidhülle von 30 bis 50 nm schwankt.

10. Verfahren zur Herstellung eines Materials so wie in einem der vorangehenden Ansprüche definiert, **dadurch gekennzeichnet, dass** es mindestens die folgenden Schritte aufweist:
1) Herstellen einer Fettphase in flüssigem Zustand, umfassend ein kristallisierbares Öl in flüssigem Zustand mit einer Schmelztemperatur T_{F} unter 100 °C, und ausgewählt aus den Fetten und den Fettgemischen natürlichen oder synthetischen Ursprungs mit einem Schmelzpunkt über 15 °C, mindesten eine Substanz von Interesse und superparamagnetische Nanopartikel, die durch mindestens eine Fettsäure funktionalisiert sind;
2) Inkontaktversetzen der Fettphase in flüssigem Zustand von Schritt 1) mit einer wässrigen Phase (PA), die zuvor auf eine Temperatur T_{PA} gebracht wurde, so dass T_{PA} höher als T_{F} ist, wobei die wässrige Phase (PA) kolloidale feste Partikel enthält;
3) Unterziehen des flüssigen Gemischs als Ergebnis von Schritt 2) einem mechanischen Rühren, um eine Öl-in-Wasser-Emulsion (O/W-Emulsion) zu erhalten, gebildet von Fettphasentröpfchen in flüssigem Zustand, die in einer kontinuierlichen wässrigen Phase dispergiert sind und in der die kolloidalen festen Partikel an der Schnittstelle vorhanden sind, die zwischen der kontinuierlichen wässrigen Phase und den dispergierten Fettphasentröpfchen gebildet ist;
4) Ruhenlassen der O/W-Emulsion, dann diese Abkühlen auf eine Temperatur T_{O/W}, die derart ist, dass T_{O/W} unter T_{F} ist, um die Verfestigung der Fettphase zu bewirken und eine O/W-Emulsion zu erhalten, die von Fettphasenkügelchen in festem Zustand gebildet ist, wobei die Kügelchen in der kontinuierlichen wässrigen Phase dispergiert sind;
5) Bilden einer Hülle, die mindestens ein Siliciumoxid um jedes der Kügelchen umfasst, durch Hinzufügen, in die kontinuierliche wässrige Phase, der O/W-Emulsion von Schritt 4), und unter mechanischem Rühren mindestens eines Siliciumoxidvorläufers, eines Tensids TA₁ und einer ausreichenden Menge von mindestens einer Säure, um die wässrige Phase auf einen pH unter oder gleich 4 zu bringen, um das Material zu erhalten;
6) eventuell Separieren des Materials von der wässrigen Phase.

11. Verfahren nach Anspruch 10, **dadurch gekennzeichnet, dass** die kolloidalen festen Partikel aus den Siliciumoxid-Nanopartikeln ausgewählt sind.

12. Verfahren nach Anspruch 10 oder 11, **dadurch gekennzeichnet, dass** die kolloidalen festen Partikel auf der Oberfläche funktionalisiert sind, um sie hydrophober zu gestalten, durch Adsorption von Molekülen eines Tensids TA₂ auf ihrer Oberfläche durch elektrostatische Bindungen.

13. Verfahren nach einem der Ansprüche 10 bis 12, **dadurch gekennzeichnet, dass** das mechanische Rühren von Schritt 3) mit Hilfe eines Dispersionsgeräts und/oder mit Hilfe eines Hochdruck-Mikroverflüssigers durchgeführt wird.

14. Material wie in einem der Ansprüche 1 bis 9 definiert oder erhalten nach dem Verfahren wie in einem der Ansprüche 10 bis 13 definiert für seine Verwendung für die magnetisch stimulierte Freisetzung mindestens einer Substanz von Interesse.

15. Verwendung eines Material wie in einem der Ansprüche 1 bis 9 definiert oder erhalten nach dem Verfahren wie in einem der Ansprüche 10 bis 13 definiert auf dem Gebiet der medizinischen Bildgebung, insbesondere als Kontrastmittel oder zur magnetischen Führung zu einem bestimmten Organ oder einem bestimmten Tumor.

## Claims

1. Material in the form of solid particles containing a solid fatty phase at the storage temperature of said material and a continuous shell comprising at least one silicon oxide and trapping said fatty phase, said fatty phase comprising:
- a crystallizable oil having a melting point (T_{M}) lower than 100 °C, and being selected from among fatty materials and mixtures of fatty materials of natural or synthetic origin having a melting point higher than 15 °C; and
- at least one substance of interest,
said storage temperature being at all times lower than the melting point of said crystallizable oil; and
said material being **characterized in that** it is sub-micrometric and **in that** the fatty phase further comprises superparamagnetic nanoparticles functionalized on the surface by at least one fatty acid.

2. The material according to claim 1, **characterized in that** the crystallizable oil is selected from among paraffins, triglycerides, fatty acids, rosins, waxes, hydrogenated vegetable oils and mixtures thereof, and synthetic bitumens.

3. The material according to claim 1 or 2, **characterized in that** the diameter of the particles varies from 400 nm to 900 nm.

4. The material according to any of the preceding clams, **characterized in that** the constituent particles of said material are monodisperse.

5. The material according to any of the preceding claims, **characterized in that** the diameter of the superparamagnetic nanoparticles contained in the fatty phase varies from 10 to 20 nm.

6. The material according to any of the preceding claims, **characterized in that** the superparamagnetic nanoparticles are nanoparticles of maghemite.

7. The material according to any of the preceding claims, **characterized in that** the fatty acid is selected from among arachidic acid, stearic acid, oleic acid, palmitic acid, myristic acid, lauric acid, capric acid and caprylic acid.

8. The material according to any of the preceding claims, **characterized in that** functionalized superparamagnetic nanoparticles represent from 0.2 to 3 weight % of the total weight of the fatty phase.

9. The material according to any of the preceding claims, **characterized in that** the thickness of the silica shell varies from 30 to 50 nm.

10. Method for preparing a material such as defined in any of the preceding claims, **characterized in that** it comprises at least the following steps:
1) preparing a fatty phase in the liquid state comprising a crystallizable oil in the liquid state having a melting point T_{M} lower than 100 °C and being selected from among fatty materials and mixtures of fatty materials of natural or synthetic origin having a melting point higher than 15°C, at least one substance of interest and superparamagnetic nanoparticles functionalized by at least one fatty acid;
2) contacting said fatty phase in the liquid state of step 1) with an aqueous phase (AP) previously brought to a temperature T_{AP} such that T_{AP} is higher than T_{M}, said aqueous phase (AP) containing solid colloidal particles;
3) subjecting the liquid mixture resulting from step 2) to mechanical stirring to obtain an oil-in-water emulsion (O/W) formed of droplets of fatty phase in the liquid state dispersed in a continuous aqueous phase and in which the solid colloidal particles are present at the interface formed between the continuous aqueous phase and the dispersed fatty phase droplets;
4) leaving said O/W emulsion to stand before cooling to a temperature T_{0/W} such that T_{0/W} is lower than T_{M} to cause solidification of the fatty phase and obtain an O/W emulsion formed of globules of fatty phase in the solid state, said globules being dispersed in the continuous aqueous phase;
5) forming a shell comprising at least one silicon oxide around each of said globules through the addition to the continuous phase of the O/W emulsion of step 4), and under mechanical stirring, of at least one precursor of silicon oxide, a surfactant TA₁ and a sufficient amount of at least one acid to bring the aqueous phase to a pH lower than or equal to 4 to obtained said material;
6) optionally, separating said material from the aqueous phase.

11. The method according to claim 10, **characterized in that** the solid colloidal particles are selected from among nanoparticles of silicon oxide.

12. The method according to claim 10 or 11, **characterized in that** the solid colloidal particles are functionalized on the surface so that they become hydrophobic through adsorption of molecules of a surfactant TA₂ on their surface via electrostatic bonds.

13. The method according to any of claims 10 to 12, **characterized in that** the mechanical stirring at step 3) is performed using dispersion apparatus and/or using a highpressure microfluidizer.

14. Material such as defined in any of claims 1 to 9 or obtained with a method such as defined in any of claims 10 to 13, for use thereof for the magnetically stimulated delivery of at least one substance of interest.

15. Use of a material such as defined in any of claims 1 to 9 or obtained with the method such as defined in any of claims 10 to 13, in the field of medical imaging, in particular as contrast agent or for magnetic guidance towards a targeted organ or tumour.
